**Europäisches Patentamt**

**European Patent Office**

**Office européen des brevets**

(19)

(11) Veröffentlichungsnummer: **0 024 547**
**B1**

(12) **EUROPÄISCHE PATENTSCHRIFT**

(45) Veröffentlichungstag der Patentschrift:
08.06.83

(51) Int. Cl.³: **C 12 P 7/42**

(21) Anmeldenummer: **80104346.4**

(22) Anmeldetag: **24.07.80**

(54) **Verfahren zur kontinuierlichen enzymatischen Umwandlung von wasserlöslichen alpha-Ketocarbonsäuren in die entsprechenden alpha-Hydroxycarbonsäuren.**

(30) Priorität: **25.07.79 DE 2930087**

(43) Veröffentlichungstag der Anmeldung:
**11.03.81 Patentblatt 81/10**

(45) Bekanntmachung des Hinweises auf die Patenterteilung:
**08.06.83 Patentblatt 83/23**

(84) Benannte Vertragsstaaten:
**BE CH FR GB LI NL SE**

(56) Entgegenhaltungen:
FR-A-2 033 404
FR-A-2 217 296
FR-A-2 293 489
FR-A-2 398 046
US-A-3 915 799

Applied Biochemistry and Microbiology, Bd. 14, Nr. 6, Mai 1979, S. 706-710 Plenum Press (US).

Die Akte enthält technische Angaben, die nach dem Eingang der Anmeldung eingereicht wurden und die nicht in dieser Patentschrift enthalten sind.

(73) Patentinhaber: **Degussa Aktiengesellschaft, Weissfrauenstrasse 9, D-6000 Frankfurt am Main 1 (DE)**
Patentinhaber: **Gesellschaft für Biotechnologische Forschung mbH (GBF), Mascheroder Weg 1, D-3300 Braunschweig-Stöckheim (DE)**

(72) Erfinder: **Wandrey, Christian, Prof. Dr. Dipl.-Chem., Berliner Strasse 23, D-5170 Jülich (DE)**
Erfinder: **Wichmann, Rolf, Dipl.-Chem., Merkatorstrasse 13, D-5170 Jülich (DE)**
Erfinder: **Leuchtenberger, Wolfgang, Dr. Dipl.-Chem., Geschwister-Scholl-Strasse 1, D-6454 Bruchköbel (DE)**
Erfinder: **Kula, Maria-Regina, Dr. Dipl.-Chem., Forstweg 15, D-3340 Wolfenbüttel (DE)**
Erfinder: **Bückmann, Andreas, Dipl.-Ing., Kutheweg 4, D-3300 Braunschweig-Stöckheim (DE)**

# Verfahren zur kontinuierlichen enzymatischen Umwandlung von wasserlöslichen α-Ketocarbonsäuren in die entsprechenden α-Hydroxycarbonsäuren

Die Erfindung betrifft ein Verfahren zur kontinuierlichen enzymatischen Umwandlung von wasserlöslichen α-Ketocarbonsäuren in die entsprechenden α-Hydroxycarbonsäuren, welches dadurch gekennzeichnet ist, dass man einem Membranreaktor, dessen Membran einen mittleren Porendurchmesser von 1 bis 3 nm aufweist, und der eine Lösung einer Formiatdehydrogenase, einer substratspezifischen Dehydrogenase und von 0,1 bis 10 mmol/l an ein Polyäthylenglykol mit einem durchschnittlichen Molekulargewicht zwischen 500 und 50 000 gebunden vorliegendem Nicotinamid-adenin-dinucleotid (NAD$^+$/NADH) enthält, kontinuierlich eine wässerige Lösung von 50 bis 100% der maximal löslichen Menge, jedoch nicht über 2000 mmol/l der umzusetzenden α-Ketocarbonsäure in Form eines wasserlöslichen Salzes als Substrat und von 100 bis 6000 mmol/l eines Formiats zuführt, über die Membran einen Differenzdruck von 0,1 bis 15 bar aufrechterhält und hinter der Membran kontinuierlich einen die gebildete α-Hydroxycarbonsäure enthaltenden Filtratstrom abführt.

Aus der US-A Nr. 3915799 ist bereits ein Verfahren zur kontinuierlichen enzymatischen Umwandlung von Pyruvat in Lactat in einem Membranreaktor in Gegenwart eines Coenzyms und von Lacetatdehydrogenase bekannt. Als Coenzym wird natives NADH eingesetzt, das mittels Äthanol in Gegenwart von Alkoholdehydrogenase regeneriert wird.

Aus der FR-A Nr. 2217296 ist ferner bereits ein diskontinuierlich betriebenes Verfahren zur enzymatischen Umwandlung von α-Ketocarbonsäuren in die entsprechenden α-Hydroxycarbonsäuren in Gegenwart von nativem NAD$^+$/NADH und einer substratspezifischen Dehydrogenase unter gleichzeitiger Regenerierung von NADH mittels Formiat in Gegenwart von Formiatdehydrogenase bekannt.

Aus der FR-A Nr. 2398046 ist schliesslich bereits ein diskontinuierlich betriebenes Verfahren zur enzymatischen Umwandlung von Dehydrocarnitin in Carnitin in Gegenwart von NAD$^+$/NADH und Carnitindehydrogenase unter gleichzeitiger Regenierung von NADH mittels Formiat in Gegenwart von Formiatdehydrogenase bekannt. Es ist ferner angegeben, dass das NAD$^+$/NADH an eine hochmolekulare, lösliche Substanz gebunden sein kann. Nähere Angaben über geeignete hochmolekulare Substanzen finden sich nicht.

Erst das erfindungsgemässe Verfahren erlaubt es jedoch, wasserlösliche α-Ketocarbonsäuren kontinuierlich und mit hohen Raum-Zeit-Ausbeuten in die entsprechenden α-Hydroxycarbonsäuren umzuwandeln und ist daher zu einer kostengünstigen Produktion dieser α-Hydroxycarbonsäuren anwendbar.

Als Reaktionsgefäss wird ein mit einer Ultrafiltrationsmembran ausgestatteter Membranreaktor verwendet, dessen Membran dazu dient, die eingesetzten Enzyme und das für die Umsetzung erforderliche Coenzym im Reaktor zurückzuhalten, aber das niedermolekulare Produkt und das nicht umgesetzte Substrat durchzulassen. Der Membranreaktor kann als sogenannter Flachmembranreaktor ausgebildet sein. Bei diesem Reaktortyp kann es sich beispielsweise um ein flaches zylindrisches Gefäss handeln, auf das ein mittels eines O-Ringes abgedichteter Deckel aufgesetzt ist. Zusammen mit dem O-Ring ist die flächenmässig relativ ausgedehnte flache Membran eingespannt. Der Substratstrom wird durch eine Dosierpumpe dem unterhalb der Membran liegenden Reaktionsraum zugeführt, der zweckmässigerweise mit einer Rühreinrichtung, z.B. einem Magnetrührer, ausgestattet ist. Der das Produkt enthaltende Filtratstrom verlässt der Reaktionsraum durch die Membran und eine zwecks Vermeidung von deren mechanischer Beanspruchung darüber angeordnete, mit Bohrungen versehene Platte und wird aus dem Deckel abgeführt. Ein sogenannter Hohlfaser-Membranreaktor, in dem ein Hohlfaserbündel aus Ultrafiltrationsmembranen, ein sogenanntes Hohlfasermodul, an die Stelle der Flachmembran tritt, ist dann vorteilhafter, wenn sich aufgrund der geometrischen Anordnung höhere Reynoldzahlen des Fluids parallel zur Membran und damit geringere Belegungen der Membran mit Enzymproteinen erreichen lassen. Bei diesem Reaktortyp handelt es sich beispielsweise um eine Art von Schlaufenreaktor, der aus einem Reaktionsbehälter, einer Zuwälzpumpe und dem Hohlfassermodul besteht. Der Substratstrom wird mittels einer Dosierpumpe dem Reaktionsbehälter zugeführt. In diesem wird das Reaktionsgemisch umgepumpt, wobei der Umpumpstrom im Verhältnis zum Substratstrom mindestens etwa 100:1 beträgt, um die Belegung der Hohlfasermembranen mit Enzymprotein so gering wie möglich zu halten. Der das Produkt enthaltende Filtratstrom tritt durch die Hohlfasermembranen hindurch und wird hinter diesen gesammelt und abgeführt. Für das erfindungsgemässe Verfahren werden Membranen verwendet, die einen mittleren Porendurchmesser von 1 bis 3 nm aufweisen. Geeignete Materialien für die Membranen sind beispielsweise Acetylcellulosen, Polyamide, Polysulfone oder modifizierte Polyvinylalkohole.

Der Membranreaktor enthält eine Lösung einer Formiatdehydrogenase, einer substratspezifischen Dehydrogenase und von durch Bindung an ein Polyäthylenglykol im Molekulargewicht vergrössertem NAD$^+$/NADH. Die Formiatdehydrogenase wird zweckmässig in einer solchen Menge eingesetzt, dass ihre Aktivität mindestens 12 000 µmol/l × min beträgt. Nach oben sollte ihre Einsatzmenge zweckmässigerweise so begrenzt werden, dass die Proteinkonzentration maximal etwa 20 g/l beträgt. Die substratspezifische Dehydrogenase wird zweckmässig in einer solchen Menge eingesetzt, dass das Verhältnis der Aktivitäten von Formiatdehydrogenase und substratspezifischer Dehydrogenase zwischen 1:1 und 1:5 liegt.

Das beim erfindungsgemässen Verfahren als Coenzym erforderliche $NAD^+$/NADH muss durch Bindung an ein wasserlösliches Polyäthylenglykol in seinem Molekulargewicht soweit vergrössert sein, dass es zwar noch wasserlöslich ist, um eine homogene Katalyse zu erlauben, andererseits aber zusammen mit den beiden Enzymen durch die Membran sicher zurückgehalten wird. Hierfür muss das wasserlösliche Polyäthylenglykol ein durchschnittliches Molekulargewicht zwischen 500 und 50 000, vorzugsweise zwischen 1500 und 10 000, aufweisen. Die Herstellung des im Molekulargewicht vergrösserten Coenzyms kann beispielsweise so erfolgen, dass man das Coenzym in seiner oxydierten Form zunächst am N(1)-Atom mit einem Alkylierungsmittel umsetzt, das eine weitere funktionelle Gruppe einführt, welche die Kupplung an das Polyäthylenglykol ermöglicht. Als solche Alkylierungsmittel kommen beispielsweise Halogencarbonsäuren, wie Jodessigsäure, Epoxycarbonsäuren, wie 3,4-Epoxybuttersäure, Lactone, wie β-Propiolacton, oder Aziridine, wie Äthylenimin, in Betracht. Das erhaltene N(1)-Derivat wird anschliessend mit Hilfe der Carbodiimidmethode [vgl. Cuatrecanas, „J. Biol. Chem.", *245*, 3059 (1970)] an das wasserlösliche Polyäthylenglykol gekuppelt, in das erforderlichenfalls vorher mit dem N(1)-Derivat reaktionsfähige Gruppen, z.B. Carboxylgruppen, eingeführt worden sind. Das erhaltene Kupplungsprodukt wird dann zum entsprechenden NADH-Derivat reduziert, durch eine Dimroth-Umlagerung in das N(6)-Derivat umgewandelt und gegebenenfalls wieder zum entsprechen den $NAD^+$-Derivat oxydiert. Das im Molekulargewicht vergrösserte Coenzym wird in einer solchen Menge eingesetzt, dass die Konzentration an $NAD^+$/NADH 0,1 bis 10 mmol/l, vorzugsweise 1 bis 7 mmol/l, beträgt.

Dem Membranreaktor wird kontinuierlich eine wässerige Lösung des Substrats und von Formiationen zugeführt. Die Konzentration des Substrats soll 50 bis 100% der maximal möglichen Konzentration betragen, darf aber nicht über 2000 mmol/l, vorzugsweise nicht über 1000 mmol/l, sein. Die Konzentration an Formiationen liegt zwischen 100 und 6000 mmol/l, vorzugsweise zwischen 300 und 2000 mmol/l. Als Formiate werden bevorzugt Natrium- oder Kaliumformiat verwendet.

Im Reaktionsraum des Membranreaktors vor der Membran wird die eingesetzte α-Ketocarbonsäure in Gegenwart der substratspezifischen Dehydrogenase und der reduzierten Form des Coenzyms (NADH) zu der entsprechenden α-Hydroxycarbonsäure reduziert, wobei das Coenzym in die oxydierte Form ($NAD^+$) übergeführt wird. Gleichzeitig wird aber durch die anwesenden Formiationen in Gegenwart der Formiatdehydrogenase ständig wieder die reduzierte Form des Coenzyms (NADH) regeneriert, wobei die Formiationen zu Kohlendioxid oxydiert werden.

Während der Umsetzung muss über die Membran ein Differenzdruck von 0,1 bis 15 bar, vorzugsweise von 0,2 bis 3 bar, aufrechterhalten werden, was durch Verwendung einer entsprechend dimensionierten Dosierpumpe für die zuzuführende Substratlösung und gegebenenfalls durch ein Drosselventil im Filtratstrom hinter der Membran erreicht wird. Der Differenzdruck bewirkt, dass ein Filtratstrom mit der gewünschten Geschwindigkeit durch die Membran hindurchtritt. Der Absolutdruck auf der Druckseite der Membran sollte zweckmässigerweise so eingestellt werden, dass auch bei kräftigem Rühren oder Umpumpen im Reaktionsraum vor der Membran zur Erzeugung einer kräftigen Turbulenz längs der Membran und damit zur Vermeidung einer Belegung der Membran mit den Enzymen oder dem im Molekulargewicht vergrösserten Coenzym an keiner Stelle der Druck so weit abgesenkt wird, dass es zu einem Entgasen des Reaktionsgemisches auf der Druckseite kommt. Der Membranreaktor wird auf einer für enzymatische Umsetzungen üblichen Temperatur zwischen 25 und 50° C gehalten. Desgleichen wird der pH des Reaktionsgemisches während der Umsetzung in dem für enzymatische Umsetzungen üblichen Bereich zwischen 5 und 9 gehalten.

Für die Durchführung des erfindungsgemässen Verfahrens geeignete Formiatdehydrogenasen können beispielsweise aus *Candida boidinii* oder aus *Pseudemonas oxalaticus* isoliert werden. Beispiele für beim erfindungsgemässen Verfahren verwendbare substratspezifische Dehydrogenasen sind L-Lactatdehydrogenase und D-Lactatdehydrogenase. Mit ihrer Hilfe kann beispielsweise Brenztraubensäure in L- bzw. D-Milchsäure, Phenylbrenztraubensäure in L- bzw. D-Phenylmilchsäure, 2-Oxo-4-methylvaleriansäure in L- bzw. D-2-Hydroxy-4-methylvaleriansäure, 2-Oxo-3-methylvaleriansäure in L- bzw. D-2-Hydroxy-3-methylvaleriansäure, 2-Oxo-3-methylbuttersäure in L- bzw. D-2-Hydroxy-3-methylbuttersäure oder 2-Oxovaleriansäure in L- bzw. D-2-Hydroxyvaleriansäure umgewandelt werden. Zweckmässigerweise werden die umzusetzenden α-Ketocarbonsäuren in Form ihrer Natrium- oder Kaliumsalze eingesetzt.

Da bei der Umwandlung von α-Ketocarbonsäuren in die entsprechenden α-Hydroxycarbonsäuren ein optisch aktives Zentrum neu gebildet wird, lässt sich die Produktkonzentration im Filtratstrom mit Hilfe eines Polarimeters kontinuierlich messen. Die gebildete α-Hydroxycarbonsäure kann aus dem Filtrat in an sich bekannter Weise gewonnen werden. Dies kann beispielsweise dadurch geschehen, dass man zur Trennung der α-Hydroxycarbonsäure von der nicht umgesetzten α-Ketocarbonsäure mittels eines basischen Ionenaustauschers die unterschiedliche Säurestärke ausnutzt. Die unterschiedliche Löslichkeit von Salzen, insbesondere der Calciumsalze, ermöglicht in vielen Fällen eine Trennung durch fraktionierte Kristallisation. Gegebenenfalls kann auch die unterschiedliche Polarität zur Trennung durch Extraktion mit einem geeigneten Lösungsmittel herangezogen werden.

Im nachfolgenden Beispiel wird das erfindungs-

gemässe Verfahren anhand der Umwandlung des Natriumsalzes der Phenylbrenztraubensäure in D-Phenylmilchsäure näher erläutert:

*Beispiel:*

Ein auf einer Temperatur von 25° C gehaltener Flachmembranreaktor mit einem Volumen von 10 ml, der mit einem Magnetrührer und einer Ultrafiltrationsmembran von 62 mm Durchmesser mit einer nominellen Ausschlussgrenze von 5000 (Lieferfirma: Amicon, Witten; Typ DM 5) ausgestattet war, wurde zur Sterilisation mittels einer auf eine Fördergeschwindigkeit von 4 ml/h eingestellten Dosierpumpe ca. 20 h lang mit wässeriger Formaldehydlösung durchgespült. Anschliessend wurde während weiterer ca. 20 h die Formaldehydlösung durch destilliertes Wasser verdrängt. Danach wurde, ebenfalls mit einer Fördergeschwindigkeit von 4 ml/h ca. 10 h lang eine über ein Sterilfilter (0,2 μ) filtrierte Substratlösung zugeführt, die 100 mmol/l Natriumphenylpyruvat und 200 mmol/l Natriumformiat sowie 100 mmol/l Natriumphosphat als Puffer enthielt und mit Natronlauge auf pH 7 eingestellt war. Darauf wurden anstelle der Substratlösung 2,5 ml einer Coenzymlösung zudosiert, die 4 mmol/l NADH, gebunden an ein Polyoxyäthylen mit einem mittleren Molekulargewicht von 10 000, und 50 mmol/l eines Phosphatpuffers für pH 7 enthielt. Nach der vollständigen Zugabe der Coenzymlösung wurde weiter die obige Substratlösung mit einer Fördergeschwindigkeit von 4 ml/h zugeführt. Dann wurden dem Reaktionsraum vor der Membran durch eine seitliche Bohrung 29,63 mg Formiatdehydrogenase (Aktivität 2,70 μmol/mg × min bei Formiat als Substrat, 25° C und pH 7) in Form einer wässerigen Glycerinlösung (50 Gewichtsprozent Glycerin; 10 mg Formiatdehydrogenase/ml) und 0,8 mg D-Lactatdehydrogenase (Aktivität 100 μmol/mg × min bei Phenylpyruvat als Substrat, 25° C und pH 7) in Form einer wässerigen Ammoniumsulfatlösung (3,2 mol/l $(NH_4)_2$ $SO_4$; 5 mg D-Lactatdehydrogenase/ml) mittels einer Spritze zugesetzt. Bei der gewählten Einstellung betrug das Verhältnis der Aktivitäten von Formiatdehydrogenase und D-Lactatdehydrogenase 1:1. Der Umsatz wurde kontinuierlich mit Hilfe einer in den Filtratstrom eingebauten Polarimeterdurchflussküvette verfolgt. Der Differenzdruck über die Membran betrug zu Beginn 1,0 bar, stieg allmählich auf 1,7 bar an und blieb dann konstant. Innerhalb einer Betriebszeit von insgesamt ca. 160 h wurden 8,36 mmol D-Phenylmilchsäure erhalten. Die maximale Umsatzrate betrug 0,068 mmol D-Phenylmilchsäure/h.

## Patentansprüche

1. Verfahren zur kontinuierlichen enzymatischen Umwandlung von wasserlöslichen α-Ketocarbonsäuren in die entsprechenden α-Hydroxycarbonsäuren, dadurch gekennzeichnet, dass man einem Membranreaktor, dessen Membran einen mittleren Porendurchmesser von 1 bis 3 nm aufweist, und der eine Lösung einer Formiatdehydrogenase, einer substratspezifischen Dehydrogenase und von 0,1 bis 10 mmol/l an ein Polyäthylenglykol mit einem durchschnittlichen Molekulargewicht zwischen 500 und 50 000 gebunden vorliegendem Nicotinamidadenindinucleotid ($NAD^+$/NADH) enthält, kontinuierlich eine wässerige Lösung von 50 bis 100% der maximal löslichen Menge, jedoch nicht über 2000 mmol/l der umzusetzenden α-Ketocarbonsäure in Form eines wasserlöslichen Salzes als Substrat und von 100 bis 6000 mmol/l eines Formiats zuführt, über die Membran einen Differenzdruck von 0,1 bis 15 bar aufrechterhält und hinter der Membran kontinuierlich einen die gebildete α-Hydroxycarbonsäure enthaltenden Filtratstrom abführt.

2. Verfahren nach Anspruch 1, dadurch gekennzeichnet, dass man die Formiatdehydrogenase und die substratspezifische Dehydrogenase in solchen Mengen einsetzt, dass das Verhältnis ihrer Aktivitäten zwischen 1:1 und 1:5 liegt.

## Claims

1. A process for the continuous enzymatic conversion of water-soluble α-ketocarboxylic acids into the corresponding α-hydroxycarboxylic acids, characterised in that an aqueous solution of from 50 to 100% of the saturation concentration, but not more than 2000 mmol/l, of the α-ketocarboxylic acid to be converted, in the form of a water-soluble salt as substrate, and from 100 to 6,000 mmol/l of a formate are continuously delivered to a membrane reactor, the membrane of which has an average pore diameter of from 1 to 3 nm, and which contains a solution of a formatedehydrogenase, and in that, a substrate-specific dehydrogenase and from 0.1 to 10 mmol/l of nicotinamideadeninedinucleotide ($NAD^+$/NADH) bound to a polyethyleneglycol having an average molecular weight of from 500 to 50,000, a differential pressure of from 0.1 to 15 bar is maintained over the membrane and a filtrate flow containing the α-hydroxycarboxylic acid which has ofrmed is continuously removed downstream of the membrane.

2. A process according to claim 1, characterised in that the formate dehydrogenase and the substrate-specific dehydrogenase are used in quantities such that the ratio of their activities is from 1:1 to 1:5.

## Revendications

1. Procédé pour la transformation enzymatique continue d'acides α-cétocarboxyliques solubles à l'eau en acides α-hydroxycarboxyliques correspondants, caractérisé en ce que, dans un réacteur à membrane dont la membrane présente un diamètre moyen de pores de 1 à 3 nm, et qui contient une solution d'une formiatedéhydro- génase, une déhydrogénase spécifique au substrat et 0,1 à 10 mmol/l de nicotinamideadénine-dinucléotide (NAD⁺/NADH), qui se présente combinée avec un polyéthylèneglycol avec un poids moléculaire moyen de 500 à 50 000, l'on fournit, continuellement, une solution aqueuse de 50 à 100% de la quantité maximale soluble, sans dépasser toutefois 2000 mmol/l, de l'acide α-céto-carboxylique que l'on doit transformer, sous forme de sel soluble à l'eau, comme substrat, et de 100 à 6000 mmol/l d'un formiate, en ce que l'on maintient sur la membrane une pression différentielle de 0,1 à 15 bar, après quoi la membrane évacue continuellement un courant de filtrat contenant l'acide α-hydroxycarboxylique formé.

2. Procédé suivant la revendication 1, caractérisé en ce que l'on met en œuvre la formiate-déhydrogénase et la déhydrogénase spécifique au substrat dans des proportions telles que le rapport entre leurs activités se situe entre 1:1 et 1:5.